# EUROPEAN PATENT APPLICATION

(11) **EP 1 437 098 A1**
(43) Date of publication of application: **14.07.2004**
(21) Application number: 02798752.8
(22) Date of filing: 13.09.2002
(51) Int. Cl.: A61B 17/68

(54) **BONE FIXING DEVICE FOR CRANIAL SURGERY**

(30) Priority: 17.09.2001 ES 200102075; 30.07.2002 ES 200101791
(71) Applicant: Gilete Garcia, Vicente, 08006 Barcelona (ES)
(72) Inventor: Gilete Garcia, Vicente, 08006 Barcelona (ES)
(74) Representative: SUGRANES - VERDONCES - FERREGÜELA
(86) International application number: PCT/ES2002/000429
(87) International publication number: WO 2003/024342

(57) **Abstract**

The device comprises a body of elastic characteristics with two end zones and an intermediate connection zone of same, one of the end zones having an aperture for its incorporation in the edge of the bone segment or bone flap separated in the operation, whereas the other end has cap means over the rest of the cranium, in order to limit the dropping of the bone segment in its new placement in the aperture made in the cranium and the intermediate part of the device being that which joins the two ends, an elastic part transversally which thereby remains housed in the separating interstice between the separated bone segment and the cranium and which permits the device to adjust to the width of the interstice made in the cutting operation in the surgical operation.

## Description

The present invention is aimed at disclosing a bone fixation device for cranial surgery which presents numerous advantages regarding the known art.

It is well-known that after a craniotomy and the resulting brain surgery, the bone element that has been separated in order to have access to the part to be operated on, which is also known as "bone flap", must again be placed and fixed in the cranium. The reason for this is not just cosmetic, but is also to improve the safety of the brain and thus, the patient. The visible bone defects can cause psychosocial rejection and also the danger of permanent damage of a mechanical nature in the brain.

Thus, the fixation systems of the bone element separated in the operation must not only ensure fixation, but also permit a bone growth-healing that avoids pseudoarthrosis.

Formerly, the fixation of the bone element separated in the operation was carried out using wires, and subsequently, by non-reabsorbable bone fixations.

Mechanically, bone fixations are not secure enough as regards their fixing power to the cranium because there is just one low-degree friction force connection. For this reason, frequent problems have been observed, such as bone flap dislocations, with depressions or protrusions from this.

Afterwards, cranial fixation miniplates appeared, which, even though they improved the quality of the fixation, were cumbersome to place.

A system known as CranioFix has also been developed consisting of two concave disks, which are assembled on a fine shaft that joins both disks when they are brought together by means of an applicator. The basic advantage of this system is that it is designed in such a way that it can cover the trephine hole. However, placement is also difficult and can give rise to complications of an epidural bleeding nature, dural tears and so on.

In order to solve the drawbacks of the previously known systems, the inventor has carried out research to develop a new fixation device for the bone element separated during the operation (craniotomy) which acts on the spongy part of the cranial bone by exerting pressure on the longitudinal direction of this bone, as if it were a "spring" or "wedge" between the bone flap of the craniotomy and the cranium.

Essentially, the device object of the invention, presents a clip-like structure, provided with elastic features and which has fixating means in the edge of the bone element separated in the operation, and for its support in the opposite edge of the cranium from which said bone or "flap" element of the craniotomy has been separated. In a preferred embodiment, the device will be made up of a clip made of fine, thin titanium plate or other metal alloys, or even synthetic materials, which present an appreciably U-shaped structure intended for inserting into the edge of the bone element separated in the operation and which, at the other end, has a tab intended for resting on the opposite part of the cranium, said end zones being joined by an intermediate zone with elastic features, for example, a V-shaped or similar structure. In this way, the device can be well fixated in the bone element, or "bone flap" separated in the operation and is thereby arranged end to end over the opposite end of the aperture made in the cranium, in order to prevent, in so far as is possible, the vertical movement of said bone element.

The number of bone fixation devices used in a craniotomy operation will vary, usually being evenly distributed along the periphery of the bone flap.

The bone fixation device object of the invention offers the advantages of carrying out the craniotomy support in a non-traumatic way, in the sense that it is not necessary to make new perforations in the bone (both of the craniotomy and of the rest of the cranium bone), nor is it necessary that it takes up the cranium epidural space, which can cause dural tears and/or epidural haematomas. It is equally resistant to pressure and does not permit dislocations of the flap. Also, it permits the placement, and subsequent bone regeneration-growth, in the space made by the craniotomy.

Furthermore, the advantage of the device object of the present invention is its great speed and simplicity of placement as regards the currently disclosed systems. In the same way, in the event of a new operation, it can be withdrawn easily since its elastic mechanism or "spring" features mean that it can be separated by a simple lever action with the minimum of effort and thus make possible its subsequent re-use.

According to an additional embodiment of the present invention, the bone fixation device is made up of a single laminar element, exclusively for cutting and bending operations, as well as mechanical or other types of treatment in order to achieve greater roughness in certain areas. Therefore, this bone fixation device comprises a generally "U"-shaped element whose intermediate section and part of one of the two parallel tabs have a spacious aperture or window, the material of which has partly separated in the form of an angled side, which determines a step opposite the "U" shaped zone, intended for resting on the cranium bone into which the aperture has been performed.

The horizontal support surfaces with the separated bone segment and with the upper face of the edge of the aperture made in the cranium have a special finish for achieving greater fixation and enhanced osseointegration in the corresponding bone parts, having thus preferably been achieved, in accordance with the improvements herein, by means of microteeth.

Several representative drawings of preferred embodiments of the present invention are hereby attached for greater understanding, by way of explanatory, but non-limiting example.
Fig. 1 shows in diagrammatic form the combination of elements in a craniotomy operation with the bone fixation device of the present invention.
Figure 2 shows the first stage of the craniotomy diagrammatically.
Figure 3 shows, in diagrammatic form, the bone flap placement operation by means of the bone fixation device object of the present invention.
Figures 4 and 5 both show details of the device object of the invention assembled after a craniotomy.
Fig. 6 shows a plan view of a coupling zone of the device.
Figures 7 to 11 show different embodiments, by way of example, of the bone fixation device object of the present invention.
Figure 12 shows a perspective view of a bone fixation device which incorporates the enhancements of the present invention.

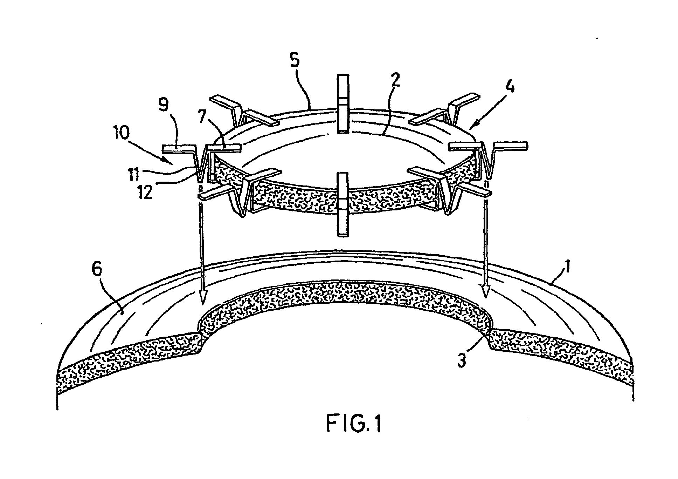

As can be observed in Figure 1, the device object of the present invention is intended in its application for a cranial operation in which, from the cranium -1-a fragment or "bone flap" -2- is separated after making the aperture determining the face of cut -3-. For the replacement of the bone fragment -2- in the previously made aperture, for the purposes of permitting the subsequent bone fixation, the present invention envisages the embodiment of a bone fixation device -4- with elastic features, having means for its smooth fixating in the edge - 5- of the bone fragment -2- and for establishing a cap over the upper surface -6-of the cranium next to the aperture -3-.

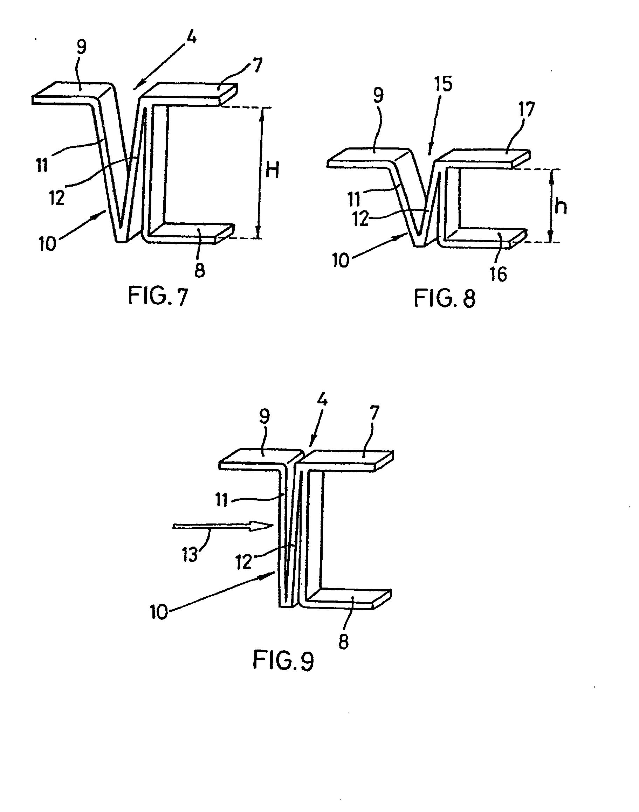

As represented in Figures 7 to 11, the device -4- will preferably present a fixation zone in the edge -5- that will preferably comprise two appreciably U-like parallel arms -7- and -8- intended for clamping the edge of the bone fragment, as has been represented in Figures 1, 3, 4 and 5, said device -4- also comprising, in the opposite end to said coupling zone by means of the arms -7- and -8-, a tab - 9- intended for establishing contact and thereby serving as a cap for arranging the bone fragment -2- appreciably at the same height as the zone -6- of the cranium next to the aperture of the intervention, the two ends of the device being linked by an intermediate zone -10- with elastic features, for example, by means of the elements -11- and -12- which adopt a V or other shape, with the possibility of lateral compression, as shown in Figure 9, wherein transversal pressure -13-causes the drawing together of the arms -11- and -12-, and inversely, once the device has been placed, the elastic action will tend to apply the vertical side -14-of separation of the arms -7- and -8- against the surface of the cut -3-.

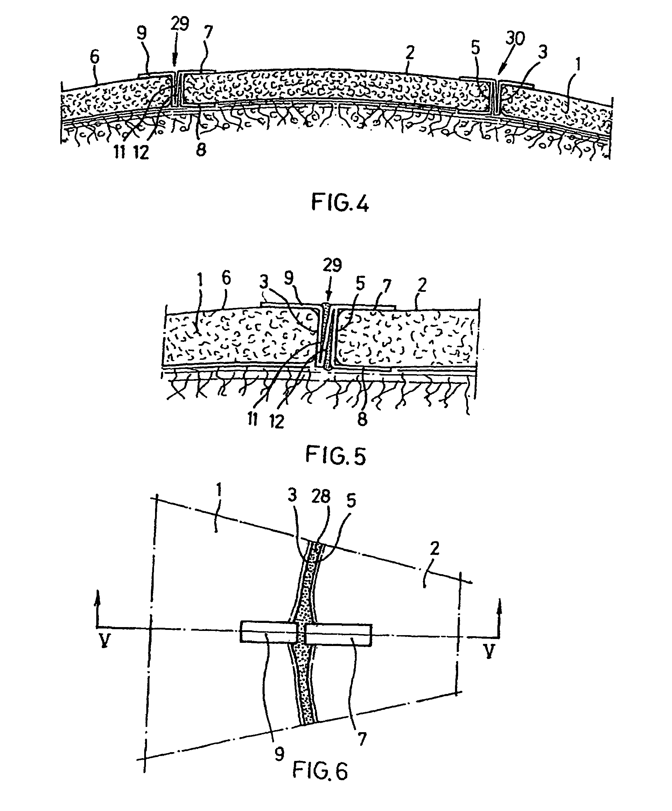

For better adaptation, it will be possible to make the device in different heights, thereby permitting the operator to select the most suitable. In Figure 8 a second element -15- has been represented in which the height h between the fixating arms -16- and -17- to the bone segment is less than the height H of the embodiment shown in Figure 4.

As is evident, what is essential to the device object of the invention is the constitution of same with elastic features, presenting one coupling end at the edge of the bone segment separated from the cranium in the intervention and another end with the possibility of a cap over the upper surface of the cranium in the vicinity of the aperture made therein, presenting, moreover, elastic features for absorbing the transversal play. As is obvious, the need to absorb said transversal play can also be justified by the possible variability of the interstice that has occurred because of the operation between the bone element or "bone flap" -2- and the aperture -3-.

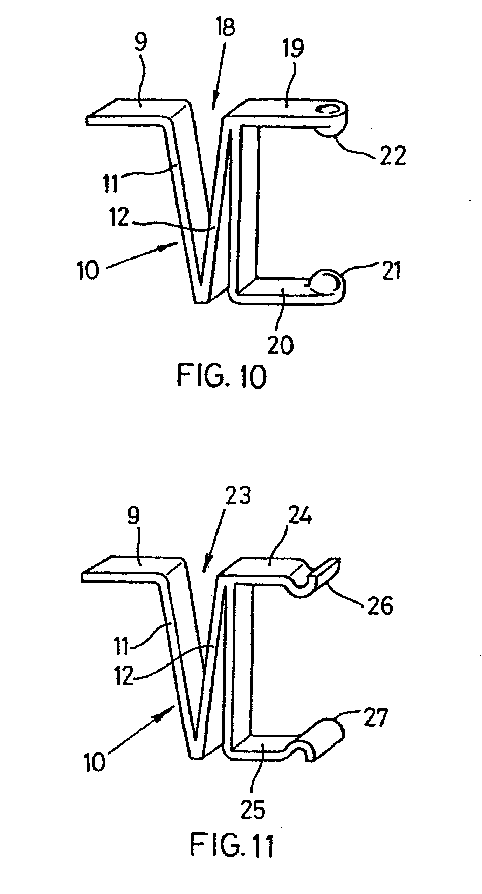

In Figures 10 and 11, embodiment examples are shown, it being observed in the case of Figure 10 that the device -18- has two arms -19- and - 20- both fitted with protuberances -21- and -22- for enhanced support on the upper and lower faces of the bone segment -2-, whereas in Figure 11, the device - 23- presents on its arms -24- and -25- bending -26- and -27- for contact with the upper and lower surface of the bone segment. These arrangements can also permit the support arms in the bone segment, such as -7- and -8- or even -19- , - 20-; -24-, -25- to exert a certain elastic action on the upper and lower faces of the bone segment, thereby permitting a better adaptation to the thickness of same.

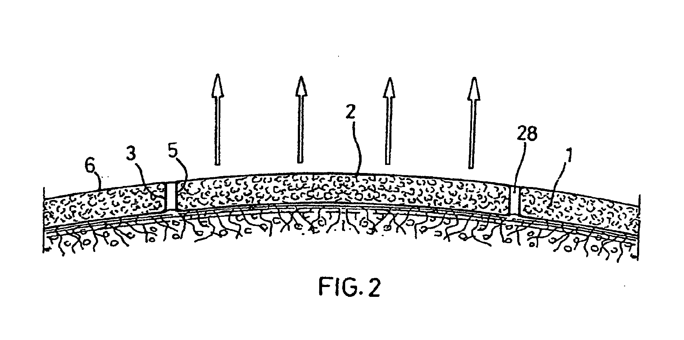

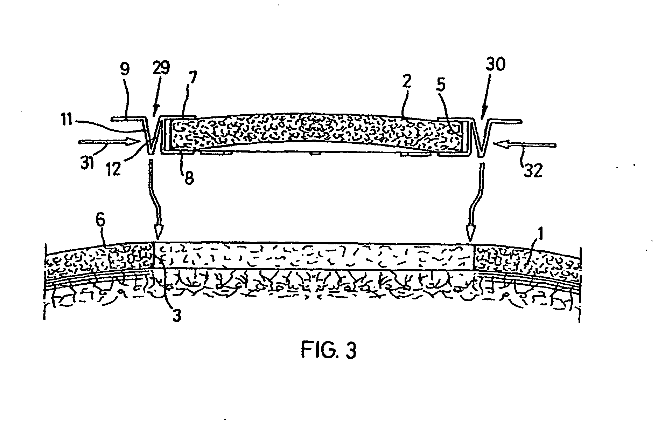

Therefore, in a craniotomy operation one will first of all proceed to separating the bone segment -2- from the rest of the cranium -1-, as shown in Figure 2, thereby producing an interstice -28- wherein the bone fixation devices shall be subsequently incorporated, as has been represented in Figures 3 to 5, wherein it is also observed that in a first stage, the fixation devices according to the present invention, such as -29- and -30-, can receive lateral pressures, represented by the vectors -31- and -32-, for permitting the coupling of the bone segment -2- in the cranium aperture, except for the interstice -28-.

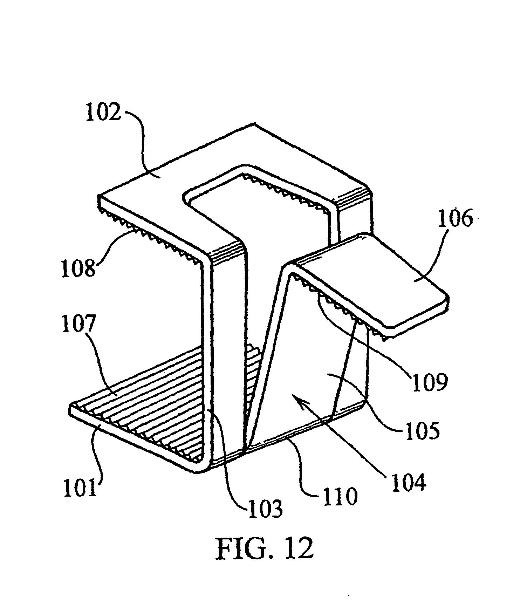

In the additional embodiment which has been shown in Figure 12, the bone fixation element presents a generally "U" structure with two parallel arms - 101- and -102- and an intermediate connection arm -103- from the first, it being a feature that the intermediate arm -103- and a good part of one of the parallel arms -102- has a large aperture wherefrom it has separated by partial cutting of the angled side -104-, and presents an arm -105- forming an acute angle with the intermediate arm from the element in "U" -103- and an upper arm bent parallel to the elements -101- and -102- from the element in "U", having been represented in the drawing with the number -106-.

By means of this set up, the same results envisaged in the main patent are achieved, clamping the edge of the bone segment separated from the cranium through the arms of the "U" -101- and -102-, and resting on the upper face of the cranium bone in which the surgical aperture has been made, the end - 106- of the angled part -104-.

The partial cut of the angled side -104-, which has been shown by way of a rectangular-shaped example, determines a connecting zone -110- with the rest of the part.

In order to improve the device's grip on the bone elements, the contact faces of elements -101- and -102- parallel to each other, indicated with the numbers -107- and -108-, as well as the lower face of the horizontal part -106-, indicated with the number -109-, have a finish which allows a greater gripping with the bone, done in any technically known way, for example, with microteeth. Nevertheless, said finish could consist of zones attacked by means of acid, or subject to sand granulating or of other elements, in order to get a finish with surface craters to obtain greater adherence, or any other known method technically appropriate to it.

## Claims

1. Bone fixation device for cranial surgery, **characterized in that** it comprises a body of elastic characteristics with two end zones and an intermediate connection zone of same, one of the end zones having an aperture for its incorporation in the edge of the bone segment or bone flap separated in the operation, whereas the other end has cap means over the rest of the cranium, in order to limit the dropping of the bone segment in its new placement in the aperture made in the cranium and the intermediate part of the device being that which joins the two ends, an elastic part transversally which thereby remains housed in the separating interstice between the separated bone segment and the cranium and which permits the device to adjust to the width of the interstice made in the cutting operation in the surgical operation.

2. Bone fixation device for cranial surgery, according to claim 1, **characterized in that** the fixating end in the bone segment separated in the operation has two arms intended for establishing contact with the upper face and the lower face next to the edge of the bone segment or bone flap.

3. Bone fixation device for cranial surgery, according to claim 2, **characterized in that** the arms appreciably parallel to each other.

4. Bone fixation device for cranial surgery, according to claim 2, **characterized in that** the arms are flexible to permit their enhanced adaptation to different thicknesses of the bone segment or bone flap.

5. Bone fixation device for cranial surgery, according to claim 3, **characterized in that** the arms have protruding zones near their ends for their enhanced adaptation over the upper and lower faces of the bone segment or bone flap.

6. Bone fixation device for cranial surgery, according to claim 1, **characterized in that** the cap of the device over the zone of the cranium next to the aperture of the operation is made up of a protruding tab.

7. Bone fixation device for cranial surgery, according to claim 1, **characterized in that** the intermediate zone between the ends of the device of elastic characteristics, adopts a V or similar shape.

8. Bone fixation device for cranial surgery, according to claim 1, **characterized in that** the constitution of the device is by means of a single laminar element bent into a "U" shape, the intermediate arm thereof and part of one of parallel arms having a large aperture, the material of which has been partly cut, leaving a connecting zone with the rest, thereby forming a slightly inclining angular element as regards the intermediate arm of the "U" and which has an upper tab parallel to the parallel arms of the "U", intended for resting on the upper face of the aperture performed surgically in the cranium.

9. Bone fixation device for cranial surgery, according to claim 8, **characterized in that** the faces in contact with the bone of the parallel arms of the element in "U" and of the flap of the angular element have a highly adherent finish.

10. Bone fixation device for cranial surgery, according to claim 9, **characterized in that** the highly adherent finish consists of microteeth.
